# EUROPÄISCHE PATENTANMELDUNG

(11) **EP 2 298 435 A1**
(43) Veröffentlichungstag der Anmeldung: **23.03.2011**
(21) Anmeldenummer: 10172718.8
(22) Anmeldetag: 13.08.2010
(51) Int. Cl.: B01D 61/02, C07C 263/20

(54) **Abtrennung von Isocyanaten aus Isocyanatmischungen durch Membrantrennverfahren**

(30) Priorität: 20.08.2009 EP 09168269
(71) Anmelder: BASF SE, 67056 Ludwigshafen (DE)
(72) Erfinder: Kamm, Andre, 49163, Bohmte (DE); Cabrera, Andres, Bogota (CO); Staudt, Claudia, 40219, Düsseldorf (DE); Sieffert, Daniel, 40789, Monheim (DE)

(57) **Zusammenfassung**

Die vorliegende Erfindung betrifft ein Verfahren zum Abtrennen von Isocyanaten aus Isocyanatmischungen bei dem bei dem man eine Isocyanatmischung an einer für diese Isocyanatmischung semipermeablen Membran trennt.

## Beschreibung

Die vorliegende Erfindung betrifft ein Verfahren zum Abtrennen von Isocyanaten aus Isocyanatmischungen bei dem man bei dem man eine Isocyanatmischung an einer für diese Isocyanatmischung semipermeablen Membran trennt.

In der Polyurethanchemie wird häufig mit Isocyanatmischungen gearbeitet. So wird beispielsweise bei der Herstellung des technisch sehr bedeutenden Diphenylmethandiisocyanats ausgehend von Anilin, Formaldehyd und Phosgen nicht nur die Zweikernverbindung und deren Isomere sondern auch höherkernige Homologe, das sogenannte Roh-MDI, erhalten. Je nach Anwendung werden aber beispielsweise nur Zweikernverbindungen, das Monomer-MDI oder vorwiegend die höherkernigen Homologen des MDI, das Polymer-MDI eingesetzt. Dazu muss das Roh-MDI aufgetrennt werden. Dies geschieht vorzugsweise durch Destillation und ist beispielsweise in WO 2002070581 und EP 1518874 beschrieben.

Für andere Anwendungen, beispielsweise bei der Herstellung von feuchtigkeitshärtenden Polyurethansystemen, wird von Polyisocyanatprepolymeren ausgegangen. Dabei werden Polyisocyanate im Überschuss mit gegenüber Isocyanaten reaktiven Verbindungen zu Polyisocyanatprepolymeren umgesetzt. Diese Polyisocyanatprepolymere härten dann unter Einfluss von Feuchtigkeit, beispielsweise von Luftfeuchtigkeit, unter Harnstoffbildung zum fertigen Polyurethan aus. Problematisch an diesem Verfahren ist, dass durch die Umsetzung von Polyisocyanaten im Überschuss gewisse Mengen an nicht umgesetzten monomeren Isocyanaten im Polyisocyanatprepolymeren enthalten sind. Da die monomeren Isocyanate gesundheitsschädlich sind, müssen diese aus dem Isocyanatprepolymeren entfernt werden. Dies geschieht ebenfalls durch Destillation.

Nachteilig an den bekannten Destillationsverfahren zur Abtrennung von Isocyanaten ist, dass dieses Verfahren sehr energieaufwendig ist und zu einer Temperaturbelastung der Isocyanate führt. Aufgabe der vorliegenden Erfindung war es daher, ein alternatives Trennverfahren für Mischungen aus Isocyanaten zu liefern.

Diese Aufgabe wird durch ein Verfahren gelöst, bei dem man eine Isocyanatmischung an einer für diese Isocyanatmischung semipermeablen Membran trennt.

Als Isocyanatmischungen können Mischungen beliebiger Verbindungen mit Isocyanatgruppen eingesetzt werden. Vorzugsweise werden als Isocyanatmischungen Mischungen auf Basis von aliphatischen, aromatischen und/oder cycloaliphatischen Di-und/oder Polyisocyanaten sowie Polyisocyanatprepolymeren eingesetzt. Dabei werden unter Polyisocyanatprepolymeren die Umsetzungsprodukte von den genannten Isocyanaten mit Verbindungen, die gegenüber Isocyanat reaktive Gruppen aufweisen, verstanden, wobei Isocyanate und Verbindungen mit gegenüber Isocyanaten reaktiven Gruppen in einem solchen Verhältnis miteinander umgesetzt werden, dass die Isocyanatgruppen im stöchiometrischen Überschuss eingesetzt werden.

Beispiele für Verbindungen mit Isocyanatgruppen sind 2,2'-, 2,4'- und 4,4'-Diphenylmethandiisocyanat, die Mischungen aus monomeren Diphenylmethandiisocyanaten und höherkernigen Homologen des Diphenylmethandiisocyanats (Polymer-MDI), Isophorondiisocyanat (IPDI) oder dessen Oligomere, 2,4- oder 2,6-Toluylendiisocyanat (TDI) oder deren Mischungen, Tetramethylendiisocyanat oder dessen Oligomere, Hexamethylendiisocyanat (HDI) oder dessen Oligomere, Naphtylendiisocyanat (NDI) oder Mischungen daraus sowie Isocyanatprepolymere auf Basis dieser Isocyanate.

Die Herstellung von Isocyantprepolymeren ist bekannt und vielfältig beschrieben. Diese sind erhältlich, indem vorstehend beschriebene Polyisocyanate im Überschuss, beispielsweise bei Temperaturen von 30 bis 100 °C, bevorzugt bei etwa 80 °C, gegebenenfalls in Gegenwart eines bekannten Katalysators mit Verbindungen mit gegenüber Isocyanatgruppen reaktiven Gruppen zum Prepolymer umgesetzt werden. Als Verbindungen mit gegenüber Isocyanatgruppen reaktiven Gruppen werden vorzugsweise Verbindungen eingesetzt, die mindestens zwei gegenüber Isocyanaten reaktive Wasserstoffatome aufweisen. Solche Verbindungen sind in der Polyurethanchemie bekannt und umfassen beispielsweise Polyetherole, Polyesterole, Polyamine oder Polythiole. Diese weisen beispielsweise eine Funktionalität von 2 bis 8 und ein Molekulargewicht von 400 bis 12000 g/mol auf.

Polyetherole werden beispielsweise hergestellt aus Epoxiden, wie Propylenoxid und/oder Ethylenoxid, oder aus Tetrahydrofuran mit wasserstoffaktiven Starterverbindungen, wie aliphatischen Alkoholen, Phenolen, Aminen, Carbonsäuren, Wasser oder Verbindungen auf Naturstoffbasis, wie Saccharose, Sorbit oder Mannit, unter Verwendung eines Katalysators. Zu nennen sind hier basische Katalysatoren oder Doppelmetallcyanidkatalysatoren, wie beispielweise in PCT/EP2005/010124, EP 90444 oder WO 05/090440 beschrieben.

Polyesterole werden z.B. hergestellt aus Alkandicarbonsäuren und mehrwertigen Alkoholen, Polythioetherpolyolen, Polyesteramiden, hydroxylgruppenhaltigen Polyacetalen und/oder hydroxylgruppenhaltigen aliphatischen Polycarbonaten, vorzugsweise in Gegenwart eines Veresterungskatalysators. Weitere mögliche Polyole sind beispielsweise im "Kunststoffhandbuch, Band 7, Polyurethane", Carl Hanser Verlag, 3. Auflage 1993, Kapitel 3.1 angegeben.

Gegebenenfalls können als Verbindungen mit gegenüber Isocyanaten reaktiven Gruppen neben den oben beschriebenen Substanzen auch Kettenverlängerungs- und/oder Vernetzungsmittel eingesetzt werden. Dies sind vorzugsweise niedermolekulare Verbindungen mit gegenüber Isocyanaten reaktiven Verbindungen, beispielsweise Glycerin, Trimethylolpropan, Glycol und Diamine. Weitere mögliche niedermolekulare Kettenverlängerungsmittel und/oder Vernetzungsmittel sind beispielsweise im "Kunststoffhandbuch, Band 7, Polyurethane", Carl Hanser Verlag, 3. Auflage 1993, Kapitel 3.2 und 3.3.2 angegeben.

Vorzugsweise weisen die Isocyanatmischungen Isocyanate auf, die im flüssigen Aggregatszustand bei gleichen äußeren Bedingungen, wie Druck und Temperatur, unterschiedliche hydrodynamische Radien aufweisen. Besonders bevorzugt werden als Isocyanatmischungen Mischungen von 2-Kern-MDI und höherkernigen Homologen des MDI, und Isocyanatprepolymere, wobei das Polyisocyanat MDI, TDI, HDI oder IPDI ist eingesetzt.

Die eingesetzten Membranen sind gegenüber der eingesetzten Isocyanatmischung semipermeabel, das heißt, sie sind nur für einen Teil der in der Isocyanatmischung enthaltenen Isocyanate permeabel, andere Teile der Isocyanatmischung werden durch die Membran zurückgehalten. Vorzugsweise bestehen die Membranen aus einem Material, das keine gegenüber Isocyanaten reaktive Gruppen wie Hydroxy-, Amino oder Carboxygruppen aufweist und in Isocyanatmischungen nicht löslich ist. Geeignete Membranmaterialien sind beispielsweise Poly(organo)silane wie Polydimethylsiloxan, Polybutadien, Polyetheretherketon, fluorierte Polymere wie z.B. Polytetrafluorethylen, Polyolefine wie Polyethylen oder Polypropylen, Polyacrylate wie Polymethylacrylat, Polyethylacrylat, Polybutylacrylat und deren Copolymere, Polystyrol, Polycarbonat, Polyarylsulfon, Poly(ether)sulfon, Polyvinylchlorid, oder Naturkautschuk. Ferner können in einer anderen Ausführungsform der Erfindung auch Membranen auf Basis anorganischer Materialien wie Zeolithe verwendet werden. In einer bevorzugten Ausführungsform der Erfindung werden vernetzte Membranmaterialien , bevorzugt auf Basis der oben genannten Polymere, eingesetzt. Dabei kann eine Vernetzung der Membranmaterialien durch Reaktionen von funktionellen Gruppen mit Vernetzern oder durch aktinische Strahlung durchgeführt werden. In einer besonderen Ausführungsform der Erfindung erfolgt die Vernetzung des Membranmaterials durch die Reaktion von Polymeren mit gegenüber Isocyanat reaktiven Gruppen und Isocyanaten, insbesondere mit Isocyanat aus der Isocyanat Mischung erfolgen.

Die verwendeten Membranmaterialen können als Platten-, Wickel-, Kissen- oder Hohlfasermodule eingesetzt werden. Diese sind dem Fachmann bekannt und beispielsweise beschrieben in "Staude, Membranen und Membrantrennprozesse, VCH Weinheim 1992. Die Membranen können dabei einen symmetrischen oder asymmetrischen Aufbau besitzen oder als Composite auf einer anorganischen oder organischen Stützschicht aufgebracht werden.

Dabei kann es sich bei den Membranen um poröse Membranen oder um nichtporöse, dichte Membranen, sogenannte Lösungs-Diffusions-Membranen, handeln. Poröse Membranen weisen Poren auf, durch die ein Teil der in der Isocyanatmischung enthaltenen Isocyanate passieren können. Dabei spricht man von einer porösen Membran wenn ein Porendurchmesser von 1 nm überschritten wird. Bei Porengrößen von kleiner 1 nm spricht man von einer nichtporösen Lösungs-Diffusions Membran. Beispiele für Membrantrennverfahren die mit porösen Membranen arbeiten sind die Ultra- bzw. Nanofiltration.

In der Praxis wird neben dem Porendurchmesser einer Membran auch die sogenannte Cut off oder Ausschlussgrenze benutzt. Dabei definiert die Cut off oder Ausschlussgrenze die minimale Molekülmasse eines Moleküls, welches durch die Membran zurück gehalten wird. Durch die Quellung von porösen Membranen kann sich der Porendurchmesser ändern. Bei einer Lösungs-Diffusions Membran kann die Quellung dazu führen, dass sich Poren in der Trennschicht ausbilden und sich ein entsprechender Cut off einstellt. Dies wird beispielsweise bei Ebert et al in J. Membr. Sci., 285, 2006, 75-80 beschrieben. Daher werden vorzugweise Membranen verwendetet, die unter realen Trennbedingungen eine Cut-off-Grenze von vorzugsweise mindestens 20% kleiner der Molmasse des im Retentat verbleibenden Isocyanats besitzt. In einer besonders bevorzugten Ausführungsform der wird die Cut-off Grenze so gewählt, dass diese mindesten 20% kleiner als die Molmasse des im Retentat verbleibenden Isocyanats ist und aber mindestens 20% größer, vorzugsweise 50% größer, als die Molmasse des im Permeat angereicherten Isocyanats.

Derartige Membranen sind beispielsweise unter den Handelsnamen SelRo®, Hersteller Koch Membrane Sytems Inc., Starmem®, der Firma Grace oder der Firma Membrane Extrcation Technology oder oNF®, der Firma GMT Membrantechnik GmbH erhältlich.

In einer besonderen Ausführungsform wird eine Isocyanatmischung als Feed vorgelegt und mit einer für diese Isocyanatmischung semipermeablen Membran in Kontakt gebracht. Dabei tritt ein Teil der Isocyanatmischung als Permeat durch die Membran hindurch. Während des Trennverfahrens kann das Permeat kontinuierlich abgezogen werden. Vorzugsweise wird die Isocyanatmischung gerührt oder an der Membran vorbei im Kreis geführt. Das auf der Feedseite verbleibende Isocyanat der Isocyanatmischung wird dabei als Retentat bezeichnet.

Die Verwendung von porösen Membranen eignet sich vorzugsweise für die Abtrennung von Isocyanaten wie TDI, HDI, IPDI oder MDI aus Mischungen oder Prepolymeren, besonders bevorzugt zur Abtrennung von Isocyanaten aus Prepolymeren. Dabei wird üblicherweise feedseitig ein Druck zwischen Umgebungsdruck und 200 bar, bevorzugt ein Druck zwischen Umgebungsdruck und 100 bar und besonders bevorzugt ein Druck zwischen Umgebungsdruck und 50 bar, angelegt. Die Temperatur der Feedseite liegt im Regelfall zwischen 20 und 100°C. Vorzugsweise wird die Trennung durchgeführt bis im Retentat maximal 1 Gew.-% monomeres Isocyanat, vorzugsweise 0,5 Gew.-% und besonders bevorzugt 0,1 Gew.-%, enthalten ist.

In Lösungs-Diffusions-Membranen Membranen, die keine durchgängigen Poren aufweisen, sondern nur freie Volumenhohlräume mit einem Durchmesser, der wesentlich kleiner als 1 nm ist, weisen bestimmte Isocyanate aus der Isocyanatmischung, bevorzugt niedermolekulare Komponenten eine hohe Löslichkeit auf. Entsprechend dem Lösungs-Diffusions-Mechanismus diffundiert das Isocyanat mit der hohen Löslichkeit auch bevorzugt durch das Membranmaterial und kann so von dem im Membranmaterial nur wenig löslichen, höhermolekularen Isocyanaten abgetrennt werden.

Die Verwendung von Lösungs-Diffusions-Membranen eignet sich vorzugsweise für die Abtrennung von Isocyanaten wie TDI, HDI, IPDI oder MDI aus Mischungen oder Prepolymeren. Besonders bevorzugt können Lösungs-Diffusions-Membranen für die Abtrennung von niedermolekularen Isocyanaten aus Polyisocynatprepolymeren verwendet werden. Der Einsatz von Lösungs-Diffusions-Membranen erfolgt dabei im Regelfall bei Temperaturen zwischen 20°C und 100°C, bevorzugt in einem Temperaturbereich zwischen 60°C und 100°C. Feedseitig wird üblicherweise ein Druck zwischen Umgebungsdruck und 5 bar, bevorzugt zwischen 1,1 und 2,5 bar angelegt, während permeatseitig üblicherweise ein Druck von 1-500 mbar, bevorzugt zwischen 10 und 50 mbar angelegt wird. Dabei wird vorzugsweise mit einem Sweepgas-Strom gearbeitet. Dazu wird bei dem angegebenen Druck ein Gas an der Permeatseite der Membran vorbeiströmen lassen. Bei dem Gas handelt es sich vorzugsweise um ein inertes Gas, wie Stickstoff oder Helium. Vorzugsweise wird die Trennung durchgeführt bis im Retentat maximal 1 Gew.-% monomeres Isocyanat, vorzugsweise 0,5 Gew.-% und besonders bevorzugt 0,1 Gew.-%, enthalten ist.

Im Folgenden wird die Erfindung anhand von Beispielen verdeutlicht:

Anhand der nachfolgend aufgelisteten Beispiele wird gezeigt, dass sich niedermolekulare Diisocyanate aus Mischungen und Prepolymeren mittels des Verfahren Pervaporation und Nanofiltration (Ultrafiltration) unter Verwendung geeigneter Membranen abtrennen lassen.

Die Pervaporationsexperimente erfolgten in einer thermostatisierten Flachzelle. Eine Lösungs-Diffusions-Membran wurde so eingebaut, dass sie permeatseitig auf einer Edelstahlfritte auflag und feedseitig mit einem Kalretz Ring aus perfluoriertem Kautschuk abgedichtet wurde. Die Trennzelle hatte eine effektive Membranfläche von 105,68 cm². In allen Fällen wurde permeatseitig eine Probe entnommen und analysiert. Die Druckfiltrationsexperimente (Nanofiltration bzw. Ultrafiltration) wurden mit einer gerührten Druckzelle der Firma Berghoff, die für Drücke bis 100 bar geeignet ist, durchgeführt. Eine poröse Membran (Nanofiltrationsmembran; mit einer Cut-off-Grenze von etwa 1000 g/mol) wurde so angebracht, dass sie permeatseitig auf einer Edelstahlfritte auflag und feedseitig mit einer Kalretz O-Ring aus perfluoriertem Kautschuk abgedichtet wurde. Die Trennzelle hatte eine effektive Membranfläche von 35,26 cm²

### Vergleichsbeispiel 1 (Pervaporation):

In die Trennzelle wurde eine Membran aus Polymethylmethacrylat eingelegt und feedseitig mit 1,5 kg Lupranat M10® beaufschlagt und auf eine Temperatur von 70°C aufgeheizt. Lupranat M10® ist eine Mischung von 4,4-MDI und höherkernigen analogen des 4,4'-MDI. Permeatseitig wurde ein Druck von 10 mbar angelegt, feedseitig herrschte Atmosphärendruck. Nach kurzer Zeit wurde das Versagen der Membran festgestellt (Durchbruch bzw. Auflösen der Membran in Feedlösung), sodass mit dieser Membran keine Trennung möglich war.

### Beispiel 1 (Pervaporation):

In die Trennzelle wurde eine Membran aus vernetzten Polydimethylsiloxan eingelegt und feedseitig mit 1,5 kg Lupranat M10® beaufschlagt und auf eine Temperatur von 70°C aufgeheizt. Permeatseitig wurde ein Druck von 10 mbar angelegt, feedseitig herrschte Atmosphärendruck. Nachdem sich ein konstanter Fluss durch die Membran eingestellt hatte wurden feed- und permeatseitig Proben gezogen. Die Analyse des Permeates zeigte, dass sich dort ausschließlich 4,4'-MDI anreicherte. Die Analyse des Feeds zeigte eine Abreicherung von 4,4'-MDI aus der Mischung um 5%, während die höheren Oligomere des MDI um etwa 11 % angereichert wurden.

### Beispiel 2 (Pervaporation):

In einem 2 L 4-Halskolben mit Stickstoffeinleitung, Rührer, Kühler und Tropftrichter wurden 1050 g Toluol-2,4-diisocyanat vorgelegt und auf etwa 60°C erwärmt. Bei 60°C wurden 450 g eines Polyesterols basierend auf Adipinsäure, Monoethylenglycol und Butandiol mit einer OH-Zahl von 56 mg KOH/g langsam über einen Zeitraum von 30 Minuten zugegeben und 2 Stunden bei 80°C nachgerührt. Das erhaltene Prepolymere hatte einen NCO-Gehalt von 32,2 %. Das Prepolymere wurde als Feedmischung für die Trennexperimente verwendet. Als Membranmaterial diente eine Membran aus vernetztem Polydimethylsiloxan. Die Feedmischung wurde auf eine Temperatur von 50°C erwärmt. Permeatseitig wurde ein Druck von 10 mbar angelegt, feedseitig herrschte Atmosphärendruck. Nachdem sich ein konstanter Fluss durch die Membran eingestellt hatte, wurden feed- und permeatseitig Proben gezogen. Die Analyse des Permeates zeigte, dass sich dort ausschließlich Toluol-2,4-diisocyanat anreicherte. Es konnten im Permeat keine höhermolekularen Komponenten gefunden werden.

### Beispiel 3 (Pervaporation):

In einem 2 L 4-Halskolben mit Stickstoffeinleitung, Rührer, Kühler und Tropftrichter wurden 1050 g Lupranat T80, eine Mischung aus 80% Toluol-2,4-diisocyanat und 20% Toluol-2,6-diisocyanat vorgelegt und auf etwa 60°C erwärmt. Bei 60°C wurden 495 g eines Polyetherols basierend auf Propylenglycol, Propylenoxid und Ethylenoxid mit einer OH-Zahl von 29 mg KOH/g langsam über einen Zeitraum von 30 Minuten zugegeben und 2 Stunden bei 80°C nachgerührt. Das erhaltene Prepolymere hatte einen NCO-Gehalt von 31,9 %. Das Prepolymere wurde als Feedmischung für die Trennexperimente verwendet. Als Membranmaterial diente eine Membran aus vernetztem Polydimethylsiloxan. Die Feedmischung wurde auf eine Temperatur von 50°C erwärmt. Permeatseitig wurde ein Druck von 10 mbar angelegt, feedseitig herrschte Atmosphärendruck. Nachdem sich ein konstanter Fluss durch die Membran eingestellt hatte wurden Feed- und Permeatseitig Proben gezogen. Die Analyse des Permeates zeigte, dass sich dort ausschließlich Toluol-2,4-diisocyanat und Toluol-2,6-diisocyanat anreicherte. Es konnten im Permeat keine höhermolekularen Komponenten gefunden werden.

### Beispiel 4 (Nanofiltration):

In einem 2 L 4-Halskolben mit Stickstoffeinleitung, Rührer, Kühler und Tropftrichter wurden 1050 g Lupranat MI eine Mischung aus 2,4'-MDI und 4,4'-MDI vorgelegt und auf etwa 60°C erwärmt. Bei 60°C wurden 450 g eines Polyetherols basierend auf Propylenglycol und Propylenoxid mit einer OH-Zahl von 18,5 mg KOH/g langsam über einen Zeitraum von 30 Minuten zugegeben und 2 Stunden bei 80°C nachgerührt. Das erhaltene Prepolymere hatte einen NCO-Gehalt von 23,1 %. Das hergestellte Prepolymere wurde als Feedlösung für die Trennexperimente verwendet. Als Membranmaterial diente eine inerte Nanofiltrationsmembran die eine Cut-off-Grenze von etwa 1000 g/mol aufweist. Die Druckfiltration wurde bei Raumtemperatur unter Stickstoff mit unterschiedlichen Drücken betrieben. Mit zunehmendem Druck wurden ansteigende Flüsse gefunden. Tabelle 1 spiegelt diese Daten wieder.

| Stickstoffdruck (Feed) [bar] | Fluss [kg/m²h] |
|---|---|
| 5 | 2,8 |
| 10 | 6,9 |
| 15 | 8,5 |
| 20 | 8,5 |

Die Analyse des Permeats zeigte, dass sich ausschließlich 4,4-MDI und 2,4'-MDI anreicherten. Es konnten keine signifikanten Mengen an höhermolekularen Verbindungen im Permeat gefunden werden.

## Patentansprüche

1. Verfahren zum Abtrennen von Isocyanaten aus Isocyanatmischungen bei dem man eine Isocyanatmischung an einer für diese Isocyanatmischung semipermeablen Membran trennt.

2. Verfahren nach Anspruch 1, **dadurch gekennzeichnet, dass** die Isocyanatmischung erhältlich ist durch Reaktion von Polyisocyanaten im Überschuss mit Verbindungen, die gegenüber Isocyanaten reaktive Gruppen aufweisen.

3. Verfahren nach Anspruch 2, **dadurch gekennzeichnet, dass** als Polyisocyanate MDI, TDI, HDI oder IPDI eingesetzt werden.

4. Verfahren nach Anspruch 3, **dadurch gekennzeichnet, dass** die Konzentration von monomerem Isocyanat im Retentat nach der Trennung kleiner 1 Gew.-%, bezogen auf das Gesamtgewicht des Retantats, ist.

5. Verfahren nach Anspruch 1, **dadurch gekennzeichnet, dass** die Isocyanatmischung eine Mischung aus zwei- und höherkernigen Homologen des MDI ist.

6. Verfahren nach einem der Ansprüche 1 bis 5, **dadurch gekennzeichnet, dass** als Membran eine Lösungs-Diffusions-Membran eingesetzt wird.

7. Verfahren nach einem der Ansprüche 1 bis 5, **dadurch gekennzeichnet, dass** als Membran eine poröse Membran, eine Nanofiltrationsmembran oder eine Ultrafiltrationsmembran eingesetzt wird.
